# EUROPÄISCHE PATENTSCHRIFT

(11) **EP 0 516 070 B1**
(45) Veröffentlichungstag und Bekanntmachung des Hinweises auf die Patenterteilung: **06.11.1996**
(21) Anmeldenummer: 92108921.5
(22) Anmeldetag: 27.05.1992
(51) Int. Cl.: C07K 1/06, C07D 209/20

(54) **Geschützte Derivate des Tryptophans, Verfahren zu deren Herstellung und deren Verwendung**
Protected tryptophan derivatives, process to synthetize them and their use
Dérivés de tryptophane protégés, procédé pour leur synthèse ainsi que leur emploi

(30) Priorität: 31.05.1991 CH 1613/91
(43) Veröffentlichungstag der Anmeldung: 02.12.1992
(73) Patentinhaber: CALBIOCHEM-NOVABIOCHEM AG, CH-4448 Läufelfingen (CH)
(72) Erfinder: White, Peter David c/o Novabiochem, GB-Bar Hill, Cambridge CB3 8SQ (GB)
(74) Vertreter: Blum, Rudolf Emil Ernst

(56) Entgegenhaltungen:
- JOURNAL OF THE CHEMICAL SOCIETY, PERKIN TRANSACTIONS. Nr. 3, März 1988, LETCHWORTH GB Seiten 497 - 502; H M FRANZEN ET AL.: 'Preparation and 11-C labeling of a substance P analogue containing D-tryptophan in position 7 and 9'
- PEPTIDES, CHEMISTRY AND BIOLOGY. (J A SMITH AND J E RIVIER, EDS). PROCEEDINGS OF THE XII AMERICAN PEPTIDE SYMPOSIUM, CAMBRIDGE (MASS), JUNE16-21,1991. 1991, LEIDEN, ESCOM Seiten 537 - 538; P. WHITE: 'Fmoc-Trp(Boc)-OH: a new derivative for the synthesis of peptides containing tryptophan

## Beschreibung

### HINTERGRUND DER ERFINDUNG

Es ist bekannt, dass die NH-Gruppe des Indolkerns der Aminosäure Tryptophan gegenüber den Angriffen verschiedener Reagenzien empfindlich ist, insbesondere gegenüber elektrophilen Angriffen, wenn die entsprechende Gruppe als nicht geschützte, freie NH-Gruppe des Indolrings vorliegt.

Bei mehrstufigen Peptidsynthesen ist es besonders wichtig, in jeder Stufe möglichst hohe Ausbeuten zu erzielen, damit in der letzten Stufe noch ausreichende Ausbeuten an dem gewünschten Endprodukt erhalten werden. Es zeigte sich, dass bei der Synthese von Peptiden, die in ihrer Struktur mindestens eine Aminosäuregruppierung aufweisen, die Tryptophan ist, dann besonders grosse Schwierigkeiten auftreten, wenn in der Peptidstruktur weitere Schutzgruppen vorliegen, beispielsweise Seitenketten-schutzgruppen, die unter nicht sehr milden Bedingungen gespaltet werden müssen, beziehungsweise bei deren Abspaltung Produkte gebildet werden, die dazu neigen, den Indolring des Tryptophans anzugreifen. Diese Schwierigkeiten treten beispielsweise dann auf, wenn in der Peptidstruktur ausserdem noch die Aminosäure Arginin, mindestens einmal enthalten ist, weil bei der Abspaltung der die Guanidingruppe des Arginins schützenden Gruppierung auch unter milden Bedingungen unerwünscht hohe Ausbeuteverluste deshalb anzutreffen sind, weil selbst unter den milden Abspaltungsbedingungen der Indolring des Triptophans angegriffen wird.

Bisher wurden verschiedene Schutzgruppen für die NH-Gruppe des Indolringes des Tryptophans in Kombination mit verschiedenen Schutzgruppen für die Alphaaminogruppe des Tryptophans eingesetzt, um diese Schwierigkeiten zu überwinden. Bisher stand jedoch keine Kombination an Schutzgruppen für den Indolkern des Tryptophans mit Schutzgruppen für die Alphaaminogruppe des Tryptophans zur Verfügung, die zu einer ausreichenden Beständigkeit der entsprechenden Tryptophanderivate führte. Bei den bisher zu diesem Zweck eingesetzten Kombinationen an Schutzgruppen traten bei der Synthese von Dipeptiden, Polypeptiden und Proteinen drastische Ausbeuteverluste auf, wenn gegen Ende der Synthese des Dipeptides, Polypeptides oder Proteins Seitenketten-Schutzgruppen von anderen Aminosäuren, die in der Sequenz vorhanden sind, abgespaltet wurden.

Ziel der vorliegenden Erfindung war es, neue, an der Alphaaminogruppe und im Indolkern geschützte Derivate des Tryptophans zur Verfügung zu stellen, mit deren Hilfe die Synthese von Dipeptiden, Polypeptiden und Proteinen ohne drastische Verluste bei der Abspaltung von Seitenketten-Schutzgruppen möglich ist.

### BESCHREIBUNG DES STANDES DER TECHNIK

In der Veröffentlichung von E. Atherton et al. in Tetrahedron,(inkl. Tetrahedron Reports), Band 44, Nr. 3, 1988, Seiten 843 - 857, wird die Verwendung von Pentafluorphenyl-estern von Aminosäuren beschrieben, deren Alphaaminogruppe mit der 9-Fluorenyl-methyloxycarbonyl-Gruppe geschützt ist, die in der Folge als Fmoc abgekürzt wird. In dieser Veröffentlichung wird unter anderem auch ein Histidinderivat beschrieben, in welchem die NH-Gruppe des Imidazolgerüstes des Histidins mit der tert.-Butyloxycarbonyl-Gruppe geschützt ist, die in der Folge als Boc abgekürzt wird. In dieser Veröffentlichung wird also ein geschütztes Histidinderivat der folgenden Struktur Fmoc-His (Boc)-Pentafluorphenyl-ester beschrieben (siehe Seite 850, Zeile 7 von unten). Die dort beschriebenen geschützten Aminosäurederivate werden in der Festphasenpeptidsynthese eingesetzt, und in dieser Veröffentlichung wird auch die Synthese einer solchen Peptidkette erwähnt, die in ihrer Aminosäuresequenz sowohl Histidin als auch Tryptophan enthält. Das zu dieser Synthese verwendete Tryptophanderivat hat seine Alphaamino-gruppe mit Fmoc geschützt, während der Indolkern des Tryptophans ungeschützt vorliegt (siehe Seite 849, Zeilen 3-10), und dort wird auch erwähnt, dass dann, wenn das entsprechende Peptid vom Harz abgespaltet wird, auf welchem die Synthese durchgeführt wurde, nur eine Ausbeute von 37% des rohen Peptides erhalten wird, weil eine Readdition eines als Zwischenprodukt gebildeten Benzylkations an den Indolkern des in der Aminosäuresequenz auftretenden Tryptophans stattfindet. Der elektrophile Angriff an der freien NH-Gruppe des Indolkerns der Aminosäure Tryptophan wird auch durch das Formelschema (7) auf Seite 850 dieser Publikation erläutert.

In der Veröffentlichung von E. Sieber et al. in Tetrahedron Letters, Band 28, Nr. 46, Seiten 6031-6034, 1987, werden geschützte Histidinderivate beschrieben, in welchen die Alphaaminogruppe des Histidines mit Fmoc geschützt ist und die NH-Gruppe des Imidazolgerüstes des Histines mit Trityl geschützt ist. In dieser Veröffentlichung wird darauf hingewiesen, dass entsprechende Histidinderivate getestet wurden, in welchen die NH-Gruppe des Imidazolringes mit anderen Schutzgruppen geschützt ist, einschliesslich der Schutzgruppe Boc, und es wurde dabei festgestellt, dass die in dieser Weise geschützten Histidinderivate instabil sind, insbesondere dann, wenn sie mit nucleophilen Materialien in Berührung kommen (siehe Seite 6031, Zeilen 5, 6 und 7 des ersten Abschnittes).

In der Veröffentlichung von D. Yamashiro et al. im Journal of the American Chemical Society, Band 94, Nr. 8, 1972, Seiten 2855 - 2859, werden ebenfalls geschützte Histidinderivate beschrieben, in welchen der Imidazolkern des Histines mit Boc geschützt ist, und die Alphaaminogruppe des Histidines entweder ebenfalls mit Boc geschützt ist oder mit der (p-Biphenylyl)isopropyl-oxycarbonyl-Gruppe geschützt ist. Die entsprechenden geschützten Histidinderivate werden zur Herstellung von Peptidketten eingesetzt, einschliesslich einer solchen, in welcher in der Aminosäuresequenz die Aminosäure Arginin auftritt, wobei die Guanidinogruppe des Arginins mit einer Nitrogruppe geschützt ist. Histidinderivate, in welchen die NH-Gruppe des Imidazolkerns mit Boc geschützt ist, weisen jedoch keine ausreichende Beständigkeit auf (siehe die vorangegangenen Erklärungen).

In der Veröffentlichung von H. Franzén L. Grehn und U. Ragnarsson in J. Chem. Soc., Chem. Commun., 1984, Seiten 1699 und 1700, wird ein Methylester des Tryptophans beschrieben, in welchem sowohl die Alphaaminogruppe als auch die NH-Gruppe des Indolskeletts durch die tert.-Butyloxycarbonyl-gruppe geschützt ist. Dort wird auch die Umwandlung des so geschützten Methylesters des Tryptophans in das entsprechende Hydrazid beschrieben und ferner erwähnt, dass aus dem Methylester beide tert.-Butyloxycarbonyl-Schutzgruppen durch eine kurze Behandlung mit Trifluoressigsäure abgespaltet werden können, während die Methylestergruppierung erhalten bleibt. Es wurde ferner festgestellt, dass bei der Behandlung des mit zwei tert.-Butyloxycarbonyl-gruppen geschützten Tryptophanmethylesters mit 2,7-molarer Salzsäure in Dioxan bei Zimmertemperatur eine selektive Abspaltung der Schutzgruppe von der Alphaaminogruppe stattfindet, während überraschenderweise die NH-Gruppe des Indolringes weiterhin mit der tert.-Butyloxy-gruppe geschützt bleibt. Die Ausbeute bei dieser selektiven Abspaltung der Alphaamino-schutzgruppe betrug 60%, längere Umsetzungszeiten, beziehungsweise höhere Salzsäurekonzentrationen, führten jedoch zu einer Abspaltung beider tert.-Butyloxycarbonyl-Schutzgruppen.

Des weiteren wird in dieser Veröffentlichung auch die Umwandlung des Tryptophan-methylesters mit geschützter NH-Gruppe des Indolringes und freier Alphaaminogruppe in ein Dipeptid nach dem Verfahren der aktiven Ester beschrieben. Spezifisch wurde an die freie Alphaaminogruppe ein Phenylalanin gebunden, dessen Aminogruppe mit tert.-Butyloxycarbonyl geschützt ist. Schliesslich wurde dieses Dipeptid durch Anknüpfung eines Leucinmethylesters an das Carboxylende des Tryptophanrestes des Dipeptides zu einem Tripeptid verlängert und schlussendlich wurde nach der selektiven Abspaltung der tert.-Butyloxycarbonyl-Schutzgruppe von der Aminogruppe des Phenylalanines auch an dieses Ende durch eine Azidkupplungsreaktion das Hydrazid des mit zwei tert.-Butyloxycarbonyl-Schutzgruppen geschützten Tryptophans angehängt, sodass das entsprechend geschützte Tetrapeptid erhalten wurde. Die Abspaltung der Schutzgruppen aus diesem vollständig geschützten Tetrapeptid wird jedoch nicht beschrieben.

In einer Fussnote dieser Veröffentlichung wird ferner darauf aufmerksam gemacht, dass entsprechende Vorversuche darauf hinweisen, dass der mit zwei tert.-Butyloxycarbonyl-gruppen geschützte Tryptophan-methylester unter Bedingungen einer katalytischen Hydrierung über Palladiumkatalysatoren teilweise zerstört wird.

Ziel der vorliegenden Erfindung war es, neue geschützte Derivate des Tryptophans zur Verfügung zu stellen, die bei der Festphasensynthese von Dipeptiden, Polypeptiden und Proteinen eingesetzt werden können, und die die oben erwähnten Nachteile der bisher bekannten geschützten Tryptophanderivate nicht aufweisen. Insbesondere sollen die angestrebten neuen Tryptophanderivate beständig sein, wenn sie einer hydrierenden Behandlung unterworfen werfen, und sie sollen ferner auch dann beständig sein, wenn Proteinketten synthetisiert werden, in welchen weitere Aminosäuregruppierungen vorliegen, die geschützte Seitenketten aufweisen, insbesondere Reste des Arginines, in welchen die Guanidinogruppe geschützt ist, insbesondere mit einer Schutzgruppe, die Sulfonyl enthält.

Ueberraschenderweise hat es sich gezeigt, dass neue Tryptophanderivate, in welchen der Indolkern mit Boc geschützt ist, und die Alphaaminogruppe des Tryptophans mit Fmoc geschützt ist, beständig sind, wenn sie hydrierenden Bedingungen unterworfen werden. Diese Eigenschaft der Tryptophanderivate ist deshalb völlig überraschend, weil entsprechende Tryptophanderivate, in welchen die Alphaamino-schutzgruppe ebenfalls Boc ist, durch eine katalytische Hydrierung teilweise zerstört werden.

Es ist ferner überraschend, dass die neuen erfindungsgemässen Tryptophanderivate die gewünschte Beständigkeit bei der Einwirkung von nucleophilen Materialien und auch bei der Einwirkung von elektrophilen Materialien aufweisen, wenn man in Betracht zieht, dass analog aufgebaute Histidinderivate gegenüber nucleophilen Materialien instabil sind, nämlich die aus dem Stande der Technik bekannten Histidinderivate, in welchen der Imidazolkern des Histidins mit Boc geschützt ist, und die Alphaaminogruppe des Histidins mit Fmoc geschützt ist.

### BESCHREIBUNG DER ERFINDUNG

Ein Gegenstand der vorliegenden Erfindung sind neue geschützte Tryptophanderivate, die dadurch gekennzeichnet sind, dass die NH-Gruppe des Indolgerüstes des Tryptophans mit der tert.-Butyloxycarbonylgruppe (Boc) geschützt ist und die Alphaaminogruppe des Tryptophans mit der 9-Fluorenyl-methyloxy-carbonyl-schutzgruppe (Fmoc) geschützt ist, und
in welchen die Carbonsäuregruppierung dieses Tryptophanderivates in freier Form, in geschützter Form, in aktivierter Form oder gebunden als Acylgruppe an den restlichen Teil eines Moleküls vorliegt, beispielsweise eines Polymermaterials oder an die Aminogruppe einer weiteren Aminosäure eines Dipeptides, eines Polypeptides oder einer Proteinstruktur.

Gemäss einer bevorzugten Ausführungsart der vorliegenden Erfindung ist daher das geschützte Aminosäurederivat ein geschütztes Tryptophanderivat, in welchem die NH-Gruppe des Indolringes durch die tert.-Butyloxy-carbonyl-gruppe geschützt ist und die Alphaamino-gruppe des Tryptophans mit der 9-Fluorenyl-methyloxycarbonyl-gruppe geschützt ist.

Gemäss einer bevorzugten Ausführungsart liegt die Carboxylgruppe dieses geschützten Tryptophanderivates als freie Carboxylgruppe vor. Gemäss einer weiteren bevorzugten Ausführungsart der Erfindung liegt die Carboxylgruppe dieses Tryptophanderivates, gebunden an die Alphaaminogruppe einer weiteren Aminosäure einer Dipeptidstruktur, einer Polypeptidstruktur oder einer Proteinstruktur vor, wobei gemäss einer speziell bevorzugten Ausführungsart dieses Dipeptid, Polypeptid oder Protein in seiner Aminosäuresequenz mindestens eine Einheit der Aminosäure Arginin und/oder eines geschützten Arginins aufweist. Speziell bevorzugte Schutzgruppen des in dem Dipeptid, Polypeptid oder Protein vorkommenden mindestens einen geschützten Argininrestes, enthalten dabei in ihrer Struktur eine Sulphonylgruppe, vorzugsweise eine Methoxy-trimethylsulphonyl-gruppe oder eine Pentamethylchroman-sulphonyl-gruppe.

Ein weiterer Gegenstand der vorliegenden Erfindung ist ein Verfahren zur Herstellung des geschützten Tryptophanderivates mit freier Carboxylgruppe, das dadurch gekennzeichnet ist, dass man
in einem ersten Reaktionsschritt Tryptophan oder ein Salz desselben, dessen Carboxylgruppe geschützt ist, vorzugsweise in Form des Benzylesters geschützt ist, an der Alphaamino-gruppe schützt, vorzugsweise mit einer Benzyloxy-carbonyl-schutzgruppe, und dass man
in einem zweiten Reaktionsschritt die NH-Gruppe des Indolringes mit der tert.-Butyloxy-carbonylgruppe schützt, sodann
in einem dritten Reaktionsschritt diejenige Gruppe, welche die Alphaaminogruppe schützt, und diejenige Gruppe, welche die Carbonsäuregruppierung schützt, gleichzeitig abspaltet, während die NH-Gruppe des Indolgerüstes mit der tert.-Butyloxy-carbonyl-gruppe geschützt bleibt, und dass
in einem vierten Reaktionsschritt die freie Alphaaminogruppe mit der 9-Fluorenyl-methyloxycarbonyl-gruppe geschützt wird.

Gemäss einer bevorzugten Ausführungsart dieses Herstellungsverfahrens wird im ersten Reaktionsschritt das Salz des Tryptophans mit der Chlorwasserstoffsäure, dessen Carboxylgruppe als Benzylester geschützt ist, in einem Lösungsmittel in Anwesenheit einer Base mit einem Carbobenzyloxy-hydroxysuccinimidester umgesetzt, um die Alphaamino-gruppe mit der Benzyloxy-carbonyl-schutzgruppe zu schützen. Bei diesem Verfahrensschritt wird als Lösungsmittel vorzugsweise Essigsäurenitril verwendet und als Base vorzugsweise ein Amin eingesetzt, wie zum Beispiel Diisopropyläthylamin.

In der zweiten Stufe des oben erwähnten Herstellungsverfahrens wird die tert.-Butyloxy-carbonylgruppe, die als Boc abgekürzt werden kann, in das aus der ersten Stufe gewonnene Zwischenprodukt eingeführt, indem man dieses mit einem entsprechenden Anhydrid der Formel

(Boc)₂O

umsetzt. Dabei wird die Umsetzung zweckmässigerweise in Anwesenheit eines Lösungsmittels durchgeführt, vorzugsweise in Essigsäurenitril, und vorzugsweise in Anwesenheit einer Base, wobei auch hier als Base Amine bevorzugt sind. Als Beispiel für ein entsprechendes Amin sei das 4-Dimethyl-aminopyridin genannt.

In der dritten Stufe des oben beschriebenen Herstellungsverfahrens wird vorzugsweise die Gruppe, welche die Carbonsäuregruppierung des Tryptophans schützt, und die Gruppe, welche die Alphaaminogruppe des Tryptophans schützt, gleichzeitig abgespaltet, während die Boc-Schutzgruppe, welche die NH-Gruppe des Indolkernes des Tryptophans schützt, bei diesen Abspaltungsreaktionen erhalten bleibt. Wie bereits erwähnt wurde, wird das Herstellungsverfahren vorzugsweise so durchgeführt, dass bei dem aus der zweiten Reaktionsstufe gewonnenen Zwischenprodukt diejenige Gruppe, welche die Carboxylgruppe des Tryptophans schützt, eine Benzylestergruppierung ist und diejenige Gruppe, welche die Alphaaminogruppe des Tryptophans schützt, eine Benzyloxycarbonyl-Schutzgruppe ist. Diese beiden Schutzgruppen können in der dritten Reaktionsstufe des Herstellungsverfahrens gleichzeitig durch eine hydrierende Spaltung unter Einsetzung eines Edelmetallkatalysators, beispielsweise Palladium, abgespalten werden.

Dementsprechend wird die dritte Reaktionsstufe des Herstellungsverfahrens vorzugsweise durchgeführt, indem man das aus der zweiten Reaktionsstufe gewonnene Zwischenprodukt, bei welchem die Alphaaminogruppe des Tryptophans mit einer BenzyloxycarbonylSchutzgruppe geschützt ist, und die Carboxylgruppe des Tryptophans in Form des Benzylesters geschützt ist, in einem Lösungsmittel mit einem Edelmetallkatalysator behandelt, vorzugsweise 5% Palladium auf Kohle. Die hydrierende Behandlung wird dabei vorzugsweise durchgeführt, indem man in Anwesenheit dieses Palladium-auf-Kohle Katalysators mit durchperlendem Wasserstoff behandelt, wobei als Lösungsmittel vorzugsweise ein niederer aliphatischer Alkohol, insbesondere Methanol eingesetzt wird.

Gemäss einer anderen Ausführungsart dieser dritten Reaktionsstufe wird die Behandlung mit diesem Edelmetallkatalysator in Anwesenheit einer aethylenisch ungesättigten Verbindung durchgeführt, beispielsweise in Anwesenheit von 1,4-Cyclohexadien, wobei auch hier in Anwesenheit eines Lösungsmittels gearbeitet wird, insbesondere in Anwesenheit eines niederen Alkohols, beispielsweise in Aethanol.

Wie bereits erwähnt wurde, hatte man vor der Entwicklung des erfindungsgemässen Verfahrens zur Herstellung der neuen Alphaaminosäurederivate, bei denen die NH-Gruppe des heterocyclischen Restes mit einer tert.-Butyloxycarbonylgruppe geschützt ist, festgestellt, dass ein entsprechend geschütztes Derivat des Indols, in welchem die Alphaaminogruppe ebenfalls mit der tert.-Butyloxycarbonylgruppe geschützt ist, und die Carbonsäuregruppierung in Form des Methylesters vorliegt, durch eine katalytische Hydrierung über einen Palladiumkatalysator teilweise zerstört wird (siehe die weiter vorne erwähnte Veröffentlichung von H. Franzén). Umso überraschender war es, dass die oben erwähnten Zwischenprodukte der zweiten Reaktionsstufe des erfindungsgemässen Verfahrens, in welchen die NH-Gruppe des Indolkernes des Tryptophans ebenfalls mit einer tert.-Butyloxycarbonylgruppe geschützt ist, während jedoch die Alphaaminogruppe mit der Benzyloxycarbonylgruppe geschützt ist, und die Carbonsäuregruppierung des Tryptophans als Benzylester geschützt ist, eine derartige hydrierende oder reduzierende Behandlung in Anwesenheit eines Palladiumkatalysators ohne teilweise Zerstörung überlebt und auch das so gewonnene Zwischenprodukt mit freier Alphaaminogruppe und freier Carboxylgruppe in Anwesenheit des Edelmetallkatalysators stabil bleibt.

Bei der Durchführung des erfindungsgemässen Verfahrens wird das Produkt der dritten Reaktionsstufe, in welchem die Carboxylgruppe und die Alphaaminogruppe des Tryptophans in freier Form vorliegt, während die NH-Gruppe des Indolkernes mit der tert.-Butyloxycarbonylgruppe geschützt ist, an der freien Alphaaminogruppe mit der 9-Fluorenyl-methyloxycarbonyl-gruppe geschützt. Diese Einführung der Schutzgruppe in das Produkt der dritten Reaktionsstufe erfolgt vorzugsweise indem man dieses mit dem 9-Fluorenyl-methyloxycarbonyl-hydroxysuccinimid umsetzt. Dieses Umsetzung wird vorzugsweise in einem Lösungsmittel in Anwesenheit einer Base durchgeführt. Bevorzugte Lösungsmittel sind dabei Essigsäurenitril, sowie Dioxan und als Base kann beispielsweise eine wässrige Alkalicarbonatlösung eingesetzt werden, insbesondere eine 10%-ige wässrige Natriumcarbonatlösung.

Wie bereits erwähnt wurde, führen die neuen, an der Aminogruppe geschützten Derivate von Alphaaminosäuren, in welchen die NH-Gruppe des heterocyclischen Ringes mit der tert.-Butyloxycarbonylgruppe geschützt ist, und die Alphaaminogruppe mit der 9-Fluorenyl-methyloxycarbonyl-gruppe geschützt ist, zu sehr guten Ausbeuten, wenn man diese geschützten Aminosäurederivate in entsprechenden Syntheseverfahren zur Herstellung von Dipeptiden, Polypeptiden oder Proteingerüsten eingesezt, und zwar aufgrund der Tatsache, dass entsprechende Zwischenprodukte der Syntheseverfahren eine gute Beständigkeit aufweisen und dass die erwähnte 9-Fluorenyl-methyloxycarbonyl Schutzgruppe der Alphaaminogruppe unter anderen Reaktionsbedingungen abgespaltet werden kann, als die tert.-Butyloxycarbonyl Schutzgruppe der NH-Gruppe der heterocyclischen Struktur.

Ein weiterer Gegenstand der vorliegenden Erfindung ist daher die Verwendung der neuen erfindungsgemässen, an der Aminogruppe geschützten Derivate der Alphaaminosäuren zur Herstellung von Dipeptid, Polypeptid oder Proteinstrukturen, indem man die an der Aminogruppe geschützten Derivate mit ihrer Carboxylgruppe an die Aminogruppe einer weiteren Aminosäure bindet, wobei diese weitere Aminosäure gegebenenfalls ein entsprechender Aminosäurerest eines Dipeptides, eines Polypeptides oder einer Proteinstruktur ist, oder indem man ein solches an der Aminogruppe geschütztes Derivat der heterocyclischen Aminosäure verwendet, dessen Acylgruppe an den restlichen Teil eines Moleküls oder eines Polymers gebunden ist, vorzugsweise dessen Acylgruppe an die Aminogruppe einer weiteren Aminosäure gebunden ist, die eine entsprechende Aminosäure eines Dipeptids, eines Polypeptids oder einer Proteinstruktur ist, und wobei man bei dieser Verwendung die entsprechende heterocyclische Aminosäure oder ein Molekül, das diese Aminosäure enthält, unter Ausbildung einer entsprechenden Dipeptid, Polypeptid oder Proteinstruktur einführt, wobei in dem hergestellten Produkt die NH-Gruppe der heterocyclischen Struktur der heterocyclischen Alphaaminosäure mit der tert.-Butyloxycarbonyl Gruppe geschützt ist.

Früher wurden Formylschutzgruppen und 2,2,2-Trichloräthoxycarbonylgruppen zur Schützung der NH-Gruppe des Indolgerüstes des Tryptophans bei Peptidsynthesen und Proteinsynthesen eingesetzt. Diese früher verwendeten Schutzgruppen weisen jedoch gegenüber der tert.-Butyloxycarbonyl-gruppe, die in den erfindungsgemässen geschützten Derivaten zum Schutz einer NH-Gruppe im heterocyclischen Grundgerüst entsprechender Alphaaminosäuren herangezogen werden, den Nachteil auf, dass sie unter alkalischen Bedingungen wieder abgespaltet werden, während die tert.-Butyloxycarbonyl-gruppe gegenüber solchen alkalischen Bedingungen, die im Zuge einer Peptidsynthese häufig angewandt werden, beständig ist.

Auch die Benzyloxycarbonyl-gruppe wurde bereits zum Schutze der NH-Gruppe des Indolkernes von Tryptophan herangezogen. Diese Schutzgruppe weist jedoch den Nachteil auf, dass sie nach der Beendigung einer Proteinsynthese oder Peptidsynthese unter drastischen Bedingungen abgespaltet werden muss, die zu einer Zerstörung von empfindlichen Peptiden führt.

Im Gegensatz dazu, lässt sich die tert.-Butyloxycarbonyl-schutzgruppe der NH-Gruppe des heterocyclischen Skelettes der erfindungsgemässen geschützten Tryptophanreste nach der Beendigung einer Peptidsynthese oder Proteinsynthese leicht durch Verwendung von Trifluoessigsäure abspalten, ohne dass eine Schädigung des hergestellten Polypeptides oder Proteines auftritt.

Eine spezieller Vorteil der tert.-Butyloxycarbonyl-gruppe als Schutzgruppe für die NH-Gruppe des Inkernes des Tryptophans besteht darin, dass sie die heterocyclische Gruppe gegenüber elektrophilen Angriffen schützt, die im Zuge von Proteinsynthesen auftreten können, insbesondere dann, wenn andere Schutzgruppen, beispielsweise Schutzgruppen für Seitenkettengruppen im Zuge einer Peptidsynthese oder Proteinsynthese wieder abgespaltet werden müssen.

In den letzten Jahren werden zum Schutze des Guanidinorestes von Arginin im allgemeinen solche Schutzgruppen verwendet, die in ihrer Struktur eine Sulphonylgruppierung aufweisen. Wenn dann diese Schutzgruppen abgespaltet werden, dann treten grosse Verluste auf, weil entsprechende, im heterocyclischen Rest ungeschützte Alphaaminosäuren, die ebenfalls in der Peptidstruktur oder Proteinstruktur vorhanden sind, bei dieser Abspaltungsreaktion sulphoniert werden. Diese Probleme sind insbesondere dann anzutreffen, wenn in dem Polypeptid oder Protein mindestens ein Tryptophanrest vorhanden ist, bei dem die NH-Gruppe des Indolkernes ungeschützt oder mit einer der vorhin erwähnten Schutzgruppen für den Indolkern geschützt ist.

Gemäss einer bevorzugten Ausführungsart der erfindungsgemässen Verwendung werden dementsprechend die neuen erfindungsgemässen geschützten Derivate des Tryptophans zur Herstellung von solchen Dipeptiden, Polypeptiden oder Proteinen eingesetzt, die als weitere Aminosäuregruppierung mindestens eine Gruppierung eines geschützten Arginines aufweisen. Dabei ist der Argininrest vorzugsweise mit einer solchen Schutzgruppe geschützt, die in ihrer Struktur eine Sulphonylgruppe aufweist, wie zum Beispiel die Methoxy-trimethylsulphonylgruppe oder 2,2,5,7,8-Pentamethyl-chroman-6-sulphonyl-schutzgruppe.

Bei der Verwendung der erfindungsgemässen geschützten Alphaaminosäure-derivate kann, nach der Durchführung der entsprechenden Syntheseverfahren, die tert.-Butyloxy-schutzgruppe, welche die NH-Gruppe der heterocyclischen Struktur der heterocyclischen Alphaaminosäure schützt, dann, wenn dies erwünscht ist, wieder abgespaltet werden, ohne dass dabei bedeutende Ausbeutverluste eintreten. Vorzugsweise erfolgt diese Abspaltung der tert.-Butyloxycarbonyl-schutzgruppe durch eine Behandlung mit Trifluoressigsäure.

Gemäss einer bevorzugten Ausrührungsart der erfindungsgemässen Verwendung wird also die tert.-Butyloxycarbonyl-gruppe, welche die NH-Gruppe der heterocyclischen Struktur schützt, aus der entsprechenden Polypeptidkette oder Proteinkette, welche als weitere Aminosäuren mindestens eine Gruppierung eines geschützten Arginins umfasst, am Ende der Peptidsynthese oder Proteinsynthese, abgespaltet, indem man eine Behandlung mit Trifluoressigsäure durchführt.

Es zeigte sich, dass bei der Verwendung von Trifluoressigsäure zur Abspaltung der die NH-Gruppe des heterocyclischen Systems schützenden tert.-Butyloxycarbonyl-gruppe ein unstabiles COOH-Derivat dieser heterocyclischen NH-Gruppierung gebildet wird. Bei der Aufarbeitung unter mild basischen Bedingungen und unter tiefen Temperaturen zerfällt dann dieses unstabile Derivat unter Freisetzung der NH-Gruppierung des heterocyclischen Restes.

Bei der Abspaltung der Schutzgruppe aus einem solchen Dipeptid, Polypeptid oder Protein, das in seiner Struktur, zusätzlich zu der geschützten heterocyclischen Aminosäure auch noch mindestens eine Einheit eines geschützten Argininrestes aufweist, ist es dabei vorteilhaft, dass die Arginin-schutzgruppe und die tert.-Butyloxycarbonyl-schutzgruppe der NH-Gruppierung der heterocyclischen Aminosäure, in einem einzigen Verfahrensschritt abspaltbar ist. Vorzugsweise wird diese Abspaltung durch eine Behandlung mit Trifluoressigsäure erreicht. Dabei konnte beobachtet werden, dass bei Anwesenheit eines geschützten Arginines, in welchem die Schutzgruppe des Guanidinrestes eine solche ist, die eine Sulphonylgruppe aufweist, diese Schutzgruppe abgespaltet wird, ohne dass der Heterozyklus der heterocyclischen Aminosäure angegriffen wird. Es ist anzunehmen, dass sich bei einer entsprechenden Behandlung mit der Trifluoressigsäure intermediär das unstabile COOH-Derivat der NH-Gruppe des heterocyclischen Restes bildet, sodass selbst bei einer gleichzeitigen Abspaltung der tert.-Butyloxycarbonyl-schutzgruppe von dieser NH-Gruppe und der sulphonylenthaltenden Schutzgruppe von Arginin keine Zerstörung der heterocyclischen Struktur durch einen elektrophilen Angriff ergibt.

Jedenfalls zeigte es sich, dass bei der Abspaltung der Schutzgruppen von einem geschützten Polypeptid, das in seiner Struktur sowohl ein erfindungsgemäss geschütztes Tryptophanderivat als auch mit Sulphonylgruppen enthaltenden Schutzgruppen geschützte Arginin-derivate enthielt, bei der endgültigen Abspaltung die Sulphonierung des Indolgerüstes des Tryptophans bei der Abspaltung der Schutzgruppe mit Trifluoressigsäure praktisch vollständig vermieden werden konnte. Im Gegensatz dazu, war bei Verwendung eines am Indolgerüst ungeschützten Tryptophans ein Ausmass der Sulphonierung bei dieser Abspaltungsreaktion mit Trifluoressigsäure von 40% zu beobachten.

Die Erfindung sei nun anhand von Beispielen näher erläutert. Anhand dieser Beispiele wird die Herstellung eines erfindungsgemäss geschützten Tryptophanderivates erläutert, das eine freie Carboxyl-schutzgruppe aufweist, dessen Alphaaminogruppe mit der 9-Fluorenylmethyloxy-carbonyl-gruppe geschützt und dessen NH-Gruppe des Indolgerüstes mit der tert.-Butyloxy-carbonyl-gruppe geschützt ist.

### Beispiel 1

### Herstellung eines Zwischenproduktes mit der in Form des Carbobenzyloxy-restes geschützten Alphaaminogruppe

Ein Aequivalent des Hydrochlorides des im Indolkern ungeschützten Tryptophan-derivates mit der als Benzylester geschützten Carboxylgruppe und Di-isopropyl-äthylamin wurden in Essigsäurenitril gelöst und es wurde ein Aequivalent an dem Carbobenzyloxy-hydroxysuccinimid zugesetzt. Man rührte die Mischung drei Stunden lang bei 0^{o}C und anschliessend wurde das Essigsäurenitril abgedampft. Man löste den Rückstand in Essigsäureäthylester und wusch die organische Phase nacheinander mit einmolarer wässriger Salzsäurelösung, einmolarer wässriger Natriumbicarbonatlösung, Wasser und schliesslich mit gesättigter Kochsalzlösung. Die organische Schicht wurde dann über Magnesiumsulfat getrocknet, abfiltriert und eingedampft. Das so erhaltene kristalline Produkt, in welchem die Carbonsäuregruppierung weiterhin als Benzylester geschützt war und die Alphaaminogruppe mit der Carbobenzyloxy-carbonylschutzgruppe geschützt war, während die NH-Gruppe des Indolgerüstes in freier Form vorlag, wurde ohne weitere Reinigung als Ausgangsprodukt des Beispiels 2 eingesetzt.

### Beispiel 2

### Einführung der tert.-Butyloxycarbonyl-schutzgruppe für die NH-Gruppe des Indolgerüstes

Ein Aequivalent des gemäss Beispiel 1 hergestellten Zwischenproduktes wurde in Essigsäurenitril gelöst. Die tert.-Butyloxycarbonylgruppe, abgekürzt als Boc, wurde mit Hilfe des entsprechenden Anhydrides der Formel

(Boc)₂O

eingeführt, indem man 1,1-Aequivalente dieses Anhydrides und 0,1-Aequivalente 4-Dimethylaminopyridin zusetzte und die Mischung eine Stunde lang bei 0°C rührte. Nach einer Reaktionszeit von einer Stunde zeigte die Durchführung einer Hochdruckflüssigchromatographie, dass die Umsetzung zu etwa 80% abgelaufen war. Danach wurden jede Stunde 0,1-Aequivalente des Anhydrides der oben angegebenen Formel zugesetzt, bis die Reaktion zu etwa 98% abgelaufen war. Es sei darauf hingewiesen, dass die Anwesenheit von zu viel Anhydrid der angegebenen Formel während des Ablaufens der Reaktion verhindert werden soll, weil ein Ueberschuss an diesem Anhydrid in der Reaktionsmischung zu der Bildung des gemischten Imides führen kann.

Sobald die Reaktion zu etwa 98% abgelaufen war, wurde das Lösungsmittel abgedampft, der Rückstand in Diäthyläther gelöst und die organische Lösung nacheinander mit zweimolarer wässriger Zitronensäure, einmolarer wässriger Bicarbonatlösung, gesättigter wässriger Kochsalzlösung und Wasser gewaschen. Anschliessend wurde dann die organische Phase über Magnesiumsulphat getrocknet und abgedampft. Nach dem Abdampfen erhielt man ein öliges Produkt, das zu etwa 94% rein war (überprüft mit der Hochdruckflüssigchromatographie). Dieses ölige Produkt, in welchem die NH-Gruppe des Indolgerüstes mit der tert.-Butyloxy-schutzgruppe geschützt war, während die Carbonsäuregruppierung und die Alphaaminogruppe mit den gleichen Schutzgruppen geschützt waren wie in dem Produkt des Beispiels 1, wurde ohne Reinigung im Verfahren des Beispiels 3 eingesetzt.

### Beispiel 3

### Gleichzeitige Abspaltung der Benzyloxycarbonyl-schutzgruppe und der Benzylester-schutzgruppe von der Carboxylgruppe

### Verfahrensvariante A

Ein Aequivalent des gemäss Beispiel 2 erhaltenen Zwischenproduktes wurde in Methanol gelöst und es wurde der Hydrierungskatalysater 5% Palladium auf Kohle, in einer Menge von einem Gewichtsteil pro 100 Gewichtsteilen des Ausgangsproduktes zugesetzt. Man liess durch die Mischung bei Raumtemperatur 12 Stunden Wasserstoffgas hindurchperlen. Dann wurde die Mischung durch Diatomeen-erde (das Markenprodukt der Bezeichnung Celit) filtriert, um den Palladium-auf-Kohle Katalysator zu entfernen, und man wusch zweimal mit Methanol.

Anschliessend wurde das Lösungsmittel abgedampft, wobei man das entsprechende Produkt, bei dem die NH-Gruppe des Indolkernes mit der tert.-Butyloxycarbonyl-gruppe geschützt war, während die Alphaaminogruppe und die Carbonsäure-gruppierung in freier Form vorlagen, ohne Reinigung als Ausgangsmaterial für das Verfahren gemäss Beispiel 4 einsetzte.

### Verfahrensvariante B

Ein Aequivalent des Produktes gemäss Beispiel 3 wurde in Aethanol gelöst und man setzte 5%-iges Palladium auf Kohle zu, und zwar in einer Menge von 1 Gewichtsteil pro 100 Gewichtsteilen des eingesetzten Ausgangsmateriales. Anschliessend gab man 10 Aequivalente an 1,4-Cyclohexadien zu und rührte die Mischung acht Stunden lang unter einer Stickstoffatmosphäre.

Schliesslich wurde die Mischung durch Diatomeen-erde (das Produkt Celit) filtriert, um den Palladium-auf-Kohle Katalysator zur entfernen und der Celit wurde zunächst mit Aethanol und anschliessend mit Essigsäure gewaschen. Die Versuche zeigten, dass Aethanol für die bei dieser Reaktion auftretende, durch Protonenübertragung stattfindende hydrierende Abspaltung das am besten geeignete Lösungsmittel ist, dass jedoch Aethanol das Endprodukt nicht gut löst. Deshalb war das Nachwaschen des Celits mit der Essigsäure nötig.

Schliesslich wurden aus der erhaltenen Lösung die Lösungsmittel abgedampft, wobei man das entsprechende Produkt, in welchem die NH-Gruppe des Indolkernes mit der tert.-Butyloxycarbonyl-gruppe geschützt ist, während die Alphaaminogruppe und die Carboxylgruppe in freier Form vorliegen, als weissen Feststoff erhielt. Dieses Produkt wurde ohne weitere Reinigung in das Verfahren gemäss dem nachfolgenden Beispiel 4 eingesetzt.

### Beispiel 4

### Schutz der Alphaaminogruppen mit der 9-Fluorenyl methyloxy-carbonylgruppe

Ein Aequivalent des gemäss Beispiel 3, Variante A oder Variante B erhaltenen Zwischenproduktes wurde in Essigsäurenitril gelöst und es wurden zwei Aequivalente an einer 10%-igen wässrigen Natriumcarbonatlösung zugesetzt. Dabei fiel das Ausgangsmaterial aus der Lösung aus. Anschliessend wurde ein Aequivalent an dem 9-Fluorenyl-methyloxy-carbonyl-hydroxysuccinimid zugesetzt, und man rührte die Mischung acht Stunden lang bei einer Temperatur von 0°C. Nach dieser Zeit konnte durch die Hochdruckflüssigchromatographie bewiesen werden, dass in der Reaktionsmischung kein Ausgangsprodukt mehr vorhanden war. Die Lösung war jedoch nach wie vor trüb.

Anschliessend wurde die Lösung unter Verwendung einer zweimolaren wässrigen Zitronensäurelösung angesäuert, bis ein pH-Wert von 3 erreicht wurde, und man extrahierte dann mit Diäthyläther.

Die organische Phase wurde nacheinander mit gesättigter wässrigen Kochsalzlösung und mit Wasser gewaschen und schliesslich über Magnesiumsulfat getrocknet. Nach dem Abdampfen des Lösungsmittels, erhielt man das gewünschte Endprodukt in Form eines schmutzigweissen Schaumes.

Durch die Hochdruckflüssigchromatographie konnte gezeigt werden, dass dieses Produkt eine Reinheit von 90% aufwies.

Bei der Durchführung der Verfahrensvariante gemäss Beispiel 3 B, war die Gesamtausbeute über sämtliche Reaktionsstufen (also berechnet auf das im Beispiel 1 eingesetzte Ausgangsmaterial) 75%. Bei der Durchführung der Variante A des Beispiels 3, war die Ausbeute, berechnet auf das im Beispiel 1 eingesetzte Ausgangsmaterial über sämtliche Reaktionsstufen 60%.

### Beispiel 5

### Herstellung eines Polypeptides unter Verwendung des gemäss Beispiel 4 hergestellten Tryptophan-derivates

Es wurde das Polypeptid der Formel Fmoc-Trp-Arg-Arg-Arg-Arg-Val-OH hergestellt.

In diesem Polypeptid bedeutet: Fmoc-Trp an der Alphaaminogruppe mit 9-Fluorenyl-methyloxy-carbonyl geschütztes Tryptophan. Arg bedeutet Arginin Val-OH ist Valin mit freier Carboxylgruppe.

Das Polypeptid mit der oben genannten Aminosäuresequenz wurde nach der Festphasensynthese auf einem Polystyrolharz mit Acrylamidresten auf Kieselgur durchgeführt, wobei an dieses Polystyrolharz die erste Aminosäure über einen Linker (Hydroxymethylphenoxyessigsäure) gebunden war, und die erste Aminosäure an ihrer Alphaaminogruppe mit der 9-Fluorenylmethyloxycarbonylgruppe (abgekürzt als Fmoc) geschützt war. Dieses Harz mit dem darauf befindlichen geschützten Valinrest wird als Fmoc-Val-NovaSyn-KA-Harz bezeichnet.

Die Kupplung der nachfolgenden Aminosäuren an dieses Harz der angegebenen Struktur erfolgte unter Verwendung der entsprechenden Aminosäuren, in welchem die Alphaamino-gruppe mit der 9-Fluorenyl-methyloxy-carbonylgruppe geschützt war, und die Carboxylgruppe der jeweiligen Aminosäure mit dem Benzotriazol-1-yl-oxy-tris-pyrrolidino-phosphonium-hexafluorphosphat (abgekürzt als PyBOP) aktiviert war.

Pro Gramm des Harzes waren 0,1 Millimol des an der Aminogruppe geschützten Valins gebunden, und die Synthese wurde mit einem Gramm des entsprechenden Harzes begonnen, indem man jeweils einen 2,5-fachen Ueberschuss der entsprechenden, an der Alphaaminogruppe geschützten und, wie erwähnt, aktivierten Aminosäure einsetzte.

Die eingeführten Argininreste waren ausserdem an der Guanidinoseitenkette mit 2,2,5,7,8-Pentamethylchroman-6-sulphonyl, abgekürzt als Pmc, geschützt.

Als letzte Aminosäure wurde bei dieser Herstellung des Polypeptides das erfindungsgemässe, gemäss Beispiel 4, hergestellte Tryptophan-derivat eingeführt, wobei auch bei diesem Tryptophan-derivat die freie Carboxylgruppe mit PyBOP aktiviert war.

Nach der Beendigung der Synthese folgte in einer einzigen Reaktionsstufe die Abspaltung der einen Sulphonylrest enthaltenden Seitenketten-schutzgruppen der vier Argininreste, sowie die Abspaltung der tert.-Butyloxycarbonyl-schutzgruppe der NH-Gruppierung des Indolrestes des Tryptophans, und ausserdem die Abspaltung des Peptides von dem Harz. Im Gegensatz dazu blieb bei dieser Abspaltungsreaktion die Schutzgruppe für die Alphaaminogruppe des Tryptophans, also die 9-Fluorenyl-methyloxy-carbonyl-schutzgruppe, erhalten, sodass das entsprechende Hexapeptid der oben angegebenen Struktur, erhalten wurde.

Diese gleichzeitige Abspaltung der erwähnten Schutzgruppen des Arginins, der NH-Schutzgruppe des Indolkernes des Tryptophans, und des Peptides vom Harz, erfolgte indem man das Harz mit einer Mischung aus 75 Gew.-% Trifluoressigsäure plus 20 Gew.-% Aethan-dithiol und 5 Gew.-% Wasser zwei Stunden lang behandelte.

Mit Hilfe der Hochdruckflüssigchromatographie konnte gezeigt werden, dass die erwähnte Abspaltungsreaktion mit einer Ausbeute von mehr als 95% durchführbar war, das heisst, dass der Ausbeuteverlust durch Sulphonierung der Tryptophanreste des Hexapeptides weniger als 5% betrug.

### Beispiel zu Vergleichszwecken

Die Herstellung des in Beispiel 5 beschriebenen Hexapeptides erfolgte analog wie in dem Beispiel 5 beschrieben, mit Ausnahme dessen, dass das Tryptophan als an der Alphaaminogruppe mit dem 9-Fluorenyl-methyloxycarbonyl-rest geschütztes Tryptophan eingeführt wurde, wobei jedoch die NH-Gruppe des Indolrestes ungeschützt war. Die Aktivierung der Carboxylgruppe des Tryptophans erfolgte auch hier wie im Beispiel 5, nämlich mit PyBOP.

Die gleichzeitige Abspaltung der Seitenkettenschutzgruppen des Arginins und des Produktes vom Harz erfolgte wieder in der gleichen Weise wie im Beispiel 5 beschrieben, und das erhaltene Endprodukt wurde analysiert. In diesem Falle zeigte es sich, dass bei der Abspaltungsreaktion nur eine 60%-ige Ausbeute erzielt werden konnte, weil 40% der Tryptophanrreste während der Abspaltungsreaktion durch Sulphonierung zerstört worden waren. Auch diese Ergebnisse wurden mit der Hochdruckflüssigchromatographie erhalten.

## Patentansprüche

1. An der Aminogruppe geschützte Tryptophanderivate, in welchen die NH-Gruppe des Indolgerüstes des Tryptophans mit der tert.-Butyloxycarbonylschutzgruppe (also der Boc-gruppe) geschützt ist, und die Alphaaminogruppe des Tryptophans mit der 9-Fluorenyl-methyloxy-carbonyl-gruppe, also der Fmoc-schutzgruppe geschützt ist, und in welchen
die Carbonsäuregruppierung dieses Tryptophan-derivates in freier Form, in geschützter Form, in aktivierter Form oder gebunden als Acylgruppe an den restlichen Teil eines Moleküls, beispielsweise eines Polymermateriales oder an die Aminogruppe einer weiteren Aminosäure eines Dipeptides, eines Polypeptides oder eines Proteins gebunden ist.

2. Geschütztes Tryptophanderivat gemäss Anspruch 1, dadurch gekennzeichnet, dass die Carboxylgruppe des Tryptophanes als freie oder aktivierte Carboxylgruppe vorhanden ist, beispielsweise einer Carboxylgruppe, die mit Benzo-triazol-1-yl-oxy-tris-pyrrolidino-phosphonium-hexafluorophosphat aktiviert ist.

3. Geschütztes Tryptophanderivat gemäss Anspruch 1, dadurch gekennzeichnet, dass die Carboxylgruppe dieses Tryptophanderivates mit den Linkern eines Kunstharzes oder an die Alphaaminogruppe einer weiteren Aminosäure einer Dipeptidstruktur, Polypeptidstruktur oder Proteinstruktur gebunden ist, wobei das entsprechende Dipeptid, Polypeptid oder Protein vorzugsweise in seiner Aminosäuresequenz mindestens eine Gruppierung der Aminosäure Arginin oder eines geschützten Arginins aufweist, in welchem die Schutzgruppe eine Sulphonylgruppe umfasst, wobei die Schutzgruppe des mindestens einen Argininrestes vorzugsweise eine Methoxy-trimethyl-sulphonyl-gruppe oder eine 2,2,5,7,8-Pentamethyl-chroman-6-sulphonyl-schutzgruppe ist.

4. Verfahren zur Herstellung des geschützten Tryptophan-derivats nach Anspruch 2, dadurch gekennzeichnet, dass man das entsprechende Tryptophan-derivat mit freier Carboxylgruppe herstellt, indem man
in einem ersten Reaktionsschritt Tryptophan oder ein Salz desselben, dessen Carbonsäuregruppierung geschützt ist, vorzugsweise als Benzylester, an der Alphaaminogruppe schützt, vorzugsweise indem man eine Benzyloxy-carbonyl-schutzgruppe einfügt und dass man
in einer zweiten Reaktionsstufe die NH-Gruppe des Indolkernes unter Einführung der tert.-Butyloxycarbonyl-schutzgruppe schützt und dass man
in einer dritten Reaktionsstufe diejenige Gruppe, welche die Alphaamino-gruppe und diejenige Gruppe, welche die Carbonsäuregruppierung schützt, gleichzeitig abspaltet, während die NH-Gruppe des Indolkernes mit der tert.-Butyloxycarbonyl-gruppe geschützt bleibt und schliesslich
in einer vierten Reaktionsstufe die freie Alphaaminogruppe des Tryptophans mit der 9-Fluorenyl-methyloxycarbonyl-gruppe schützt und
entweder dieses Tryptophan-derivat mit freier Carboxylgruppe isoliert oder die Carboxylgruppe des Tryptophan-derivats aktiviert.

5. Verfahren nach Anspruch 4, dadurch gekennzeichnet, dass in dem ersten Reaktionsschritt das Hydrochloridsalz des Tryptophans, dessen Carboxylgruppe als Benzylester geschützt ist, in einem Lösungsmittel in Anwesenheit einer Base mit einem Carbobenzyloxyhydroxy-succinimidester umgesetzt wird, um die Alphaaminogruppe des Tryptophans mit der Benzyloxycarbonyl-schutzgruppe zu schützen, und wobei bei dieser Umsetzung als Lösungsmittel vorzugsweise Essigsäurenitril eingesetzt wird, und als Base vorzugsweise ein Amin verwendet wird, wie zum Beispiel Diisopropyl-äthyl-amin.

6. Verfahren nach Anspruch 4 oder 5, dadurch gekennzeichnet, dass man in der zweiten Reaktionsstufe die tert.-Butyloxycarbonyl-gruppe, welche als Boc abgekürzt wird, in das Zwischenprodukt der ersten Reaktionsstufe einführt, indem man eine Umsetzung mit einem Anhydrid der Formel
(Boc)₂O
durchführt und dass man diese Einführung der tert.-Butyloxycarbonyl-schutzgruppe zum Schutze der NH-Gruppe des Indolkernes durchführt, indem man das Zwischenprodukt der ersten Reaktionsstufe mit dem genannten Anhydrid in einem Lösungsmittel in Anwesenheit einer Base umsetzt, wobei als Lösungsmittel vorzugsweise Essigsäurenitril und als Base vorzugsweise ein Amin verwendet wird, wie zum Beispiel das 4-Dimethylaminopyridin.

7. Verfahren nach einem der Ansprüche 4 bis 6, dadurch gekennzeichnet, dass in dem Produkt, das nach der Durchführung der zweiten Reaktionsstufe erhalten ist, diejenige Gruppierung, welche die Carbonsäuregruppierung des Tryptophans schützt, der Benzylester ist, und diejenige Gruppe, welche die Alphaamino-gruppe des Tryptophans schützt, die Benzyloxycarbonyl-schutzgruppe ist, und dass man in der dritten Stufe gleichzeitig die Schutzgruppe für die Alphaamino-gruppe und die Schutzgruppe für die Carbonsäure-gruppierung abspaltet, während die tert.-Butyloxycarbonyl-schutzgruppe für die NH-Gruppe des Indolkernes unangegriffen bleibt, indem man das Produkt der zweiten Reaktionsstufe in Anwesenheit eines Lösungsmittels, vorzugsweise Methanol oder Aethanol, in Anwesenheit eines Edelmetallkatalysators, vorzugsweise 5% Palladium-auf-Kohle, behandelt, wobei diese hydrierende Behandlung entweder mit Hilfe von durchperlendem Wasserstoffgas oder durch Zugabe einer aethylenisch ungesättigten Verbindung, vorzugsweise 1,4-Cyclohexadien, erfolgt.

8. Verfahren nach einem der Ansprüche 4 bis 7, dadurch gekennzeichnet, dass das Reaktionsprodukt der dritten Reaktionsstufe, welches eine freie Carboxylgruppe und eine freie Alphaamino-gruppe aufweist, während die NH-Gruppe des Indolkernes mit der tert.-Butyloxycarbonyl-gruppe geschützt ist, an der freien Alphaaminogruppe mit der 9-Fluorenyl-methyloxycarbonyl-gruppe schützt, indem man das Produkt der dritten Reaktionsstufe mit dem 9-Fluorenyl-methyloxycarboxyl-hydroxy-succinimid umsetzt, vorzugsweise in Anwesenheit eines Lösungsmittels und vorzugsweise in Anwesenheit einer Base, wobei als Lösungsmittel vorzugsweise Essigsäurenitril und als Base vorzugsweise eine 10%-ige wässrige Natriumcarbonatlösung verwendet wird.

9. Verfahren zur Herstellung des geschützten Tryptophanderivates nach Anspruch 2, dadurch gekennzeichnet, dass man im Tryptophan mit freier Carboxylgruppe und freier Alphaaminogruppe die Alphaaminogruppe schützt, vorzugsweise indem man eine Benzyloxycarbonylschutzgruppe einfügt, beispielsweise durch Umsetzung mit Carbobenzyloxychlorid und anschliessend die freie Carbonsäuregruppierung schützt, vorzugsweise als Benzylester, indem man beispielsweise eine Umsetzung mit Benzylbromid durchführt und dass man dann anschliessend die zweite bis vierte Reaktionsstufe des Verfahrens gemäss Anspruch 4 durchführt.

10. Verwendung der an der Alphaaminogruppe und an der NH-Gruppe des Indolringes geschützten Tryptophanderivate gemäss Anspruch 1, zur Herstellung von Dipeptiden oder Polypeptiden oder Proteingerüsten, indem man die an der Alphaaminogrupps mit Fmoc geschützten Tryptophanderivate über ihre Carboxylgruppe an die Aminogruppe einer weiteren Aminosäure bindet, die gegebenenfalls eine Aminosäure-gruppierung eines Dipeptides, Polypeptides oder einer Proteinstruktur ist, oder
indem man ein entsprechendes, an der Aminogruppe mit Fmoc geschütztes Tryptophanderivat verwendet, dessen Carboxylgruppe als Acylrest an den restlichen Teil eines Moleküls oder eines Polymers gebunden ist, beispielsweise an ein Kunstharz oder an die Aminogruppe einer weiteren Aminosäure eines Dipeptides, eines Polypeptides oder einer Proteinstruktur, und wobei man das entsprechende Tryptophanderivat oder ein Molekül, welches diese enthält, unter Ausbildung eines Dipeptides, eines Polypeptides oder eines Proteins einführt, wobei die NH-Gruppe des Indolkernes des Tryptophanrestes in dem entsprechenden Dipeptide, Polypeptid oder Protein, mit der tert.-Butyloxycarbonyl-gruppe geschützt ist.

11. Verwendung nach Anspruch 10, dadurch gekennzeichnet, dass die neuen Tryptophanderivate, beziehungsweise Strukturen, welche diese Derivate als Aminosäurerest enthalten, zur Herstellung von solchen Dipeptiden, Polypeptiden und Proteinen herangezogen werden, welche als weitere Aminosäureeinheit mindestens eine Einheit eines geschützten Arginins enthalten, vorzugsweise eines Arginins, in welchem die Schutzgruppe eine Sulphonylgruppierung enthält, wie zum Beispiel die Methyloxy-trimethylsulphonyl-gruppe oder die 2,2,4,7,8-Pentamethyl-chroman-6-sulphonyl-gruppe.

12. Verwendung nach Anspruch 10 oder 11, dadurch gekennzeichnet, dass nach der Herstellung der vollständigen Dipeptid-, Polypeptid- oder Proteinkette die tert.-Butyloxycarbonyl-gruppe, welche die NH-Gruppe des Indolkernes schützt, abgespaltet wird, wobei man die entsprechende freie NH-Gruppe erhält, und wobei die tert.-Butyloxycarbonyl-schutzgruppe vorzugsweise durch eine Behandlung mit Säure, insbesondere Trifluoressigsäure abgespaltet wird.

13. Verwendung nach Anspruch 12, dadurch gekennzeichnet, dass man die tert.-Butyloxycarbonyl-gruppe, welche die NH-Gruppe eines Indolkernes schützt, von dem Tryptophan eines Dipeptides, einer Polypeptidkette oder einer Proteinkette abspaltet, wobei diese mindestens eine weitere Aminosäure enthält, die ein geschütztes Arginin ist, vorzugsweise ein solches, in welchem die Schutzgruppe eine Sulfonylgruppe enthält, und dass entweder die Schutzgruppe des Arginins zunächst und anschliessend die tert.-Butyloxycarbonyl-schutzgruppe der NH-Gruppe des Tryptophanrestes abgespaltet wird oder dass die Arginin-schutzgruppe gleichzeitig mit der tert.-Butyloxycarbonyl-gruppe abgespaltet wird, beispielsweise durch eine Behandlung mit Trifluoressigsäure.

## Claims

1. Tryptophan derivatives being protected at the amino group wherein the NH-group of the indole nucleus of the tryptophan is protected by the tert.-butyloxycarbonyl protective group (i.e. the Boc-group) and the alpha-amino group of the tryptophan is protected by the 9-fluorenylmethyloxycarbonyl group, i.e. the Fmoc-protective group and wherein
the carboxylic acid group of said tryptophan derivative is present in a free form, in a protected form or in an activated form or bonded as acyl group to the remaining part of the molecule, for example to a polymeric material or to the amino group of a further amino acid of a dipeptide or of a polypeptide or of a protein.

2. Protected tryptophan derivative according to claim 1, characterized in that the carboxylic acid group of the tryptophan is present as free or as activated carboxylic acid group, for example as a carboxylic acid group activated as benzotriazole-1-yl-oxytrispyrrolidinophosphoniumhexafluoro phosphate.

3. Protected tryptophan derivative according to claim 1, characterized in that the carboxylic acid group of the tryptophan is bonded to the linkers of a resin or to the alpha-amino group of a further amino acid of a dipeptide structure, polypeptide structure or protein structure, whereby the corresponding dipeptide, polypeptide or protein displays, preferably in its amino acid sequence at least a moiety of the amino acid arginine or of a protected arginine, in which the protective group comprises a sulfonyl group, whereby the protective group of the at least one arginine group is preferably a methoxy-trimethylsulfonyl group or a 2,2,5,7,8-pentamethylchroman-6-sulfonyl protective group.

4. Process for the preparation of a protected tryptophan derivative according to claim 2, characterized in that the corresponding tryptophan derivative having a free carboxylic acid group is prepared, in which process
in a first reaction step, tryptophan or a salt thereof which has its carboxylic acid group protected, preferably as benzyl ester, is protected with respect to the alpha amino group, preferably by introducing a benzyloxycarbonyl group and that
in a second reaction step, the NH-group of the indole nucleus is protected through the introduction of the tert.-butyloxycarbonyl protective group and that
in a third reaction step, the group which protects the alpha-amino group and the group which protects the carboxylic acid group, are split off simultaneously, while the NH-group the indole nucleus remains protected by the tert.-butyloxycarbonyl group, and finally
in a fourth reaction step, the free alpha-amino group of the tryptophan is protected with the 9-fluorenylmethyloxycarbonyl group, and
either this tryptophan derivative having a free carboxylic acid acid group is isolated or the carboxylic acid group of said protected tryptophan derivative is activated.

5. Process according to claim 4, characterized in that in the first reaction step there is used the hydrochloric acid salt of the tryptophan derivative, the carboxylic acid group of which is protected as benzyl ester and said salt is reacted in a solvent and in the presence of a base with a carbobenzyloxyhydroxysuccineimide ester in order to protect the alpha-amino group of the tryptophan with the benzyloxycarbonyl-protective group and whereby the reaction is performed in the solvent acetonitrile and wherein the base which is present during said reaction is an amine, preferably a tertiary amine, like diisopropylethylamine.

6. Process according to claim 4 or 5, characterized in that in the second reaction step the tert.-butyloxycarbonyl-group which is abbreviated as Boc is introduced into the intermediate product of the first reaction step, by performing a reaction with an anhydride having the formula
(Boc)₂O
and wherein said introduction of the tert.-butyloxycarbonyl protective group is performed for the protection of the NH-group of the indole nucleus, by reacting the intermediate product of the first reaction step with said anhydride in a solvent and in presence of a base, whereby acetonitrile is preferably be used as solvent and an amine is preferably be used as a base, for example the 4-dimethylaminopyridine.

7. Process according to any of the claims 4 to 6, characterized in that in the product which is obtained after the second reaction step, the groups protecting the carboxylic acid groups of the tryptophan are benzyl esters, and the group protecting the alpha-amino group of the tryptophan is the benzyloxycarbonyl-protective group and that in the third step the protective group of the alpha-amino group and the protective group of the carboxylic acid group are split off simultaneously, while the tert.-butyloxycarbonyl-protective group of the NH-group of the indole nucleus remains untouched by subjecting the product of the second reaction step in the presence of a solvent, preferably methanol or ethanol and in the presence of a noble metal catalyst, preferably 5% palladium on carbon, to a treatment whereby said hydrogenation treatment is either performed by bubbling hydrogen gas through the reaction medium or by adding an ethylenically unsaturated compound, preferably 1,4-cyclohexadiene.

8. Process according to any of the claims 4 to 6, characterized in that the reaction product of the third reaction step which has a free carboxylic acid group and a free alpha-amino group while the the NH-group of the indole nucleus is protected with the tert.-butyloxycarbonyl-group, is protected with respect to the free alpha-amino group with the 9-fluorenyl-methyloxycarbonyl group, by reacting the product of the third reaction step with the 9-fluorenyl-methyloxycarbonylhydroxysuccinimide, preferably in the presence of a solvent and preferably in presence of a base, whereby acetonitrile is preferably used as solvent and a 10%-solution of sodium carbonate is preferably used as base.

9. Process for the preparation of a protected tryptophan derivative according to claim 2, characterized in that the alpha-amino group of the tryptophan having a free carboxylic acid group and a free amino group is protected, preferably by inserting a benzyloxycarbonyle-protective group, e.g. by reaction with carbobenzyloxy chloride and thereafter the free carboxylic acid group is protected, preferably as benzyl ester, for example by reacting with benzyl bromide and that thereafter the second to fourth reaction steps of the process according to claim 4 are performed.

10. Use of of tryptophan derivatives according to claim 1 being protected with regard to the alpha-amino group and the NH-group of the indole nucleus, for the preparation of dipeptides or polypeptides or protein systems by bonding the tryptophan derivatives protected by Fmoc to the amino group of a further amino acid by its carboxylic acid group which is optionally an amino acid group of a dipeptide, polypeptide or of a protein structure, or
by using a tryptophan derivatives being protected by Fmoc, whereby the carboxylic acid group is bonded as an acyl group to the remaining part of a molecule or of a polymer, for example to a synthetic resin or to an amino group of a further amino acid of a dipeptide, a polypeptide or of a protein structure and whereby the corresponding tryptophan derivative or a molecule which contains said molecule is introduced with the formation of a dipeptide, polypeptide or of a protein structure, whereby the NH-group of the indole nucleus in the corresponding dipeptide, polypeptide or protein structure is protected by the tert.-butyloxycarbonyl-group.

11. Use according to claim 10, characterized in that the novel tryptophan derivatives or structures respectively containing said derivatives as amino acid residues are used for the preparation of such dipeptides, polypeptides or proteins, which contain as further amino acid unit at least one further unit of a protected arginine, preferably an arginine containing a sulfonyl group as protective group, for example the methyloxytrimethylsulfonyl group or the 2,2,4,7,8-pentamethylchroman-6-sulfonyl group.

12. Use according to claim 10, characterized in that the tert.-butyloxycarbonyl group protecting the NH-group of the indole nucleus is split off after the preparation of the complete dipeptide, polypeptide or protein chain, whereby the corresponding free NH-group is obtained and whereby the tert.-butyloxycarbonyl group is split off, preferably by treatment with an acid, in particular with trifluoro acetic acid.

13. Use according to claim 12, characterized in that the tert.-butyloxycarbonyl group-protecting the NH-group of an indole nucleus is split off from a dipeptide, polypeptide or protein chain, whereby said chain contains at least one further amino acid being a protected arginine, preferably a such, in which the protective group contains a sulfonyl group and that either first the protective group of the arginine and thereafter the tert.-butyloxycarbonyl-protective group of the NH-group of the tryptophan residue is split off or that the arginine protective group is simultaneously split off with the tert.-butyloxycarbonyl group, for example by the treatment with trifluoro acetic acid.

## Revendications

1. Des dérivés de tryptophane protégés dans lesquels le radical NH du système d'indole du tryptophane est protégé par un groupe de tert.-butyloxycarbonyle (c'est-à-dire le groupe Boc) et le radical d'alpha-amino du tryptophane est protégé par le groupe 9-fluorenylmethyloxycarbonyle (c'est-à-dire le groupe Fmoc), et dans lesquels
le groupe d'acide carboxylique de ce dérivé du tryptophane est en forme libre, en forme protégée, en forme activée ou liée comme un radical d'acyle à la partie restante d'une molécule, par exemple d'un matériel polymérisé ou au radical d'amine d'un autre acide aminé d'un dipeptide, d'un polypeptide ou d'une protéine.

2. Dérivé de tryptophane protégé suivant la revendication 1, caractérisé en ce que le radical d'acide carboxylique du tryptophane peut être présent en forme de radical d'acide carboxylique libre ou activé, par exemple en forme d'un radical d'acide carboxylique étant activé par le hexafluorophosphate de benzotriazole-l-yl-oxytrispyrrolidinophosphonium.

3. Dérivé de tryptophane protégé suivant la revendication 1, caractérisé en ce que le radical d'acide carboxylique de ce tryptophane est lié aux "linkers" d'une résine synthétique ou bien au radical aminé d'un autre acide aminé d'une structure dipeptidique, polypeptidique ou protéique, alors que le dipeptide, le polypeptide ou la protéine respectifs dispose par préférence dans sa séquence d'amine aminé au moins un radical d'acide aminé qui est l'arginine ou l'arginine protégée, dans lequel le groupe protecteur renferme un groupe de sulfonyle, alors que le groupe protecteur dudit au moins radical d'arginine est par préférence un groupe de méthoxytrimethylsulfonyle ou un groupe de 2,2,5,7,8-pentamethylchroman-6-sulfonyle.

4. Procédé pour la fabrication d'un dérivé de tryptophane protégé suivant la revendication 2, caractérisé en ce que le dérivé de tryptophane respectif ayant un groupe d'acide carboxylique libre est fabriqué, en
protégeant du tryptophane ou un sel de ce dernier dans une première étape de la réaction, dont son groupe d'acide carboxylique est protégé, par préférence comme ester benzylique, sur sa position d'alpha-amino, par préférence en insérant un groupe de benzyloxycarbonyle et en
protégeant le radical NH du noyau d'indole en insérant le groupe de tert.-butyloxycarbonyle dans une deuxième étape de la réaction et en
détachant simultanément dans une troisième étape de la réaction le radical qui protège le groupe d'alpha-amino et le radical qui protège le radical d'acide carboxylique, tandis que le radical NH du noyau d'indole reste protégé par le groupe tert.-butyloxycarbonyle et, finalement, en
protégeant dans une quatrième étape de la réaction le groupe libre d'alpha-amino du tryptophane par le groupe 9-fluorenylmethyloxycarbonyle et, soit en
isolant ce dérivé de tryptophane ayant un radical d'acide carboxylique, soit en activant ledit radical d'acide carboxylique du dérivé de tryptophane.

5. Procédé suivant la revendication 4, caractérisé en ce qu'on fait réagir dans la première étape de la réaction le sel de l'hydrochlorure de tryptophane, dont le radical d'acide carboxylique est protégé comme ester benzylique par un ester de succinimide de carbobenzyloxyhydroxyl dans un solvant en présence d'une base, afin de protéger le radical d'alpha-amino du tryptophane avec le groupe protecteur de benzyloxycarbonyle et dont lors de cette réaction l'acétonitrile est utilisé par préférence comme solvant et un amine est utilisé comme base, par préférence le diisopropyléthylamine.

6. Procédé suivant la revendication 4 ou 5, caractérisé en ce qu'on fait incorporer dans la deuxième étape de la réaction le groupe tert.-butyloxycarbonyle, étant abrégé par Boc, dans le produit intermédiaire de la première étape de la réaction, en effectuant une réaction avec un anhydride de la formule
(Boc)₂O
et en effectuant cette introduction du groupe protecteur de tert.-butyloxycarbonyle afin de protéger le noyau d'indole, en faisant réagir le produit intermédiaire de la première étape de la réaction avec ledit anhydride dans un solvant en présence d'une base, alors qu'on utilise comme solvant par préférence l'acétonitrile et comme base un amine, comme par exemple le 4-diméthylaminopyridine.

7. Procédé suivant l'une des revendications 4 à 6, caractérisé en ce que dans le produit qui est obtenu après avoir effectué la deuxième étape de réaction, le groupe protégeant le radical d'acide carboxylique du tryptophane représente l'ester de benzyle, et le groupe protégeant le groupe d'alpha-amino du tryptophane représente le groupe protecteur de benzyloxycarbonyle, et qu'on sépare le groupe protecteur du groupe d'alpha-amino et le groupe protecteur du radical d'acide carboxylique au cours de la troisième étape de la réaction, tandis que le groupe protecteur de tert.-butyloxycarbonyle pour le radical NH du noyau d'indole reste intact, en traitant le produit de la deuxième étape de la réaction en présence d'un solvant, par préférence du méthanol ou de l'éthanol, en présence d'un produit catalytique de métal précieux, par préférence du palladium de 5% sur du charbon, dont ledit traitement d'hydrogénation est effectué, soit à l'aide de gaz d'hydrogène barbotant, soit par l'addition d'un composé éthylèniquement non-saturé, par préférence du 1,4-cyclohexadiène.

8. Procédé suivant l'une des revendications 4 à 7, caractérisé en ce que le produit de la troisième étape de la réaction qui dispose d'un radical d'acide carboxylique libre et un radical d'alpha-amino libre, alors que le radical NH du noyau d'indole est protégé par le groupe de tert.-butyloxycarbonyle, est protégé par un groupe de 9-fluorenylmethyloxycarbonyle en faisant réagir le produit de la troisième étape de réaction avec le succinimide d'hydroxy de 9-fluorenylmethyloxycarbonyle, par préférence en présence d'un solvant et par préférence en présence d'une base, alors que l'acétonitrile est utilisé comme solvant de préférence et une solution aqueuse de 10% de carbonate de sodium est utilisée de préférence comme base.

9. Procédé pour la fabrication du dérivé de tryptophane suivant la revendication 2, caractérisé en ce qu'on protège le radical d'alpha-amino du tryptophane ayant un radical d'acide carboxylique libre et un radical d'alpha-amino libre, par préférence en insérant un groupe protecteur de benzyloxycarbonyle, par exemple par réaction avec du chlorure de carbobenzyloxy et ensuite on protège le groupe d'acide carboxylique, par préférence comme ester de benzyle, en effectuant, par exemple une réaction avec du bromure de benzyle et en effectuant ensuite les réactions des étapes 2 à 4 du procédé suivant la revendication 4.

10. Utilisation des dérivés de tryptophane suivant la revendication 1 étant protégé du radical d'alpha-amino et du radical NH- du noyau d'indole, pour la fabrication de dipeptides ou polypeptides ou de systèmes protéiques, en attachant les dérivés de tryptophane, étant protégés du groupe d'alpha-amino par le Fmoc, d'une autre acide aminé qui représente optionellement un radical d'acide aminé d'un dipeptide, polypeptide ou d'une structure protéique, ou
en utilisant un dérivé de tryptophane étant protégé du groupe d'alpha-amino par le Fmoc, dont le radical d'acide carboxylique est lié sur la partie restante d'une molécule ou d'un polymère, par exemple à une résine synthétique ou à un groupe d'amine d'un autre acide aminé d'un dipeptide, d'un polypeptide ou d'une structure protéique et dont le dérivé de tryptophane respectif ou une molécule contenant ce dernier qui est introduit sous formation d'un dipeptide, d'un polypeptide ou d'une protéine, alors que le radical NH du noyau d'indole du radical de tryptophane est protégé par le groupe de tert.-butyloxycarbonyl dans le dipeptide, polypeptide ou protéine.

11. Utilisation suivant la revendication 10, caractérisée en ce que les nouveaux dérivés de tryptophane, respectivement les structures contenant ces dérivés comme radical d'acide aminé sont utilisés pour la fabrication de ces dipeptides, polypeptides ou protéines qui contiennent comme autre unité d'acide aminé au moins une unité d'arginine protégée, par préférence une arginine dont le groupe protecteur contient un radical de sulfonyle, comme par exemple le groupe de méthoxytriméthylsulfonyl ou le groupe de 2,2,4,7,8-pentaméthylchroman6-sulfonyle.

12. Utilisation suivant la revendication 10, caractérisée en ce qu'après la fabrication de la chaîne complète du dipeptide, du polypeptide ou de la protéine, le groupe de tert.-butyloxycarbonyle protégeant le radical de NH du noyau d'indole est séparé, alors qu'un radical libre respectif est obtenu et dont le groupe protecteur de tert.-butyloxycarbonyle est, par préférence, séparé par un traitement acide, notamment par l'acide trifluoracétique.

13. Utilisation suivant la revendication 12, caractérisée en ce que le groupe tert.-butyloxycarbonyle protégeant le radical de NH du noyau d'indole est séparé du tryptophane d'un dipeptide, d'une chaîne polypeptidique ou d'une chaîne polyprotéique, alors qu'elle contient au moins un autre acide aminé qui est un arginine protégé, par préférence un arginine dont le groupe protecteur contient un radical de sulfonyle et que, soit le groupe protecteur de l'arginine est séparé d'abord et ensuite le groupe protecteur de tert.-butyloxycarbonyle du radical de NH du radical de tryptophane est séparé, soit le groupe protecteur d'arginine est séparé simultanément avec le groupe de tert.-butyloxycarbonyle, par exemple par traitement avec de l'acide trifluoracétique.
